# EUROPEAN PATENT SPECIFICATION

(11) **EP 1 137 398 B1**
(45) Date of publication and mention of the grant of the patent: **08.10.2003**
(21) Application number: 98962686.6
(22) Date of filing: 11.12.1998
(51) Int. Cl.: A61K 9/00, A61K 31/485, A61K 9/12, A61P 11/00, A61P 25/04

(54) **PHARMACEUTICAL PREPARATION FOR INHALATION OF AN OPIOID**
PHARMAZEUTISCHE ZUBEREITUNGEN ZUR INHALATION EINES OPIOIDS
PREPARATION PHARMACEUTIQUE POUR INHALATION D'UN OPIOIDE

(43) Date of publication of application: 04.10.2001
(73) Proprietor: PHARMACHEMIE B.V., 2031 GA Haarlem (NL)
(72) Inventor: VERKERK, Volcmar, NL-1052 BN Amsterdam (NL); BLOM-ROSS, Marianne, Elisabeth, NL-2106 CH Heemstede (NL); VAN DORT, Karin, NL-1421 TK Uithoorn (NL); DE VOS, Dick, NL-2341 LP Oegstgeest (NL)
(74) Representative: van der Kloet-Dorleijn, Geertruida W.F., Drs.
(86) International application number: NL9800713
(87) International publication number: WO00035417

(56) References cited:
- WO-A-91/11179
- WO-A-92/14466
- WO-A-97/17948
- WO-A-97/35562
- WO-A-98/31352

## Description

The present invention relates to the inhalation of opioids, such as morphine, administered as a dry powder.

The pharmacologic properties of opioids include effects on the central nervous system and the bowel and include analgesia, drowsiness, changes in mood, respiratory depression, reduced gastrointestinal mobility, nausea, vomiting, and miosis.

### BACKGROUND OF THE INVENTION

Opioids are mainly used for the relief of moderate to severe pain. In addition, reports have been published on the use of opioids in the treatment of dyspnoea and neurally mediated mucus secretion.

In the treatment of pain as well as dyspnoea, opioids are administered parentally and orally. Inhalation of nebulized opioids solutions has been reported to be effective with lower doses and less side effects, as compared to the parental and oral route of administration. As nebulizers are widely used in clinical practice, morphine is frequently administered by the nebulized route. Reference is in this respect made to Farncombe M. Chater S and Gillin A, "The use of nebulized opioids for breathlessness: a chart review," Palliative Medicine 1994: 8; 306-312, and to Farncombe M and Chater S, "Clinical application of nebulized opioids for treatment of dyspnoea in patients with malignant disease," Support Care Cancer 1994: 2; 184-187.

The use of solutions for inhalation administered by a nebulizer has several drawbacks, such as escape of vapour through the mask during expiration and trapping of the nebulizer solution in the nebulizer. Also to inhale by means of a nebulizer takes some time, which can be aggravating for terminally ill patients.

Dry powder formulations for inhalation comprising morphine-HES microspheres having a high percentage of respirable particles are disclosed in WO-A-97.35562.

Dry powder inhalation compositions comprising an antitussive morphinan derivative or a salt thereof in micronized form and lactose as a carrier are disclosed in WO-A-92.14466.

Micronized dry powder formulations for inhalation comprising tramadol and lactose as carrier are disclosed in WO-A-97.17948.

### SUMMARY OF THE INVENTION

The object of the present invention is to provide a convenient and reliable method of administering opioids. More specifically, the administration is by inhalation.

The invention therefore relates to a pharmaceutical dry powder composition for inhalation consisting of micronized particles of an opioid having a fine particle fraction of at least 10%.

For administration by inhalation, the compositions according to the invention are conveniently delivered by conventional means, e.g. in the form of a single-dose premetered system such as the Cyclohaler™ using cartridges, or a premetered disposable inhaler such as the Disphaler™, or in the form of a multidose reservoir system such as the Cyclovent™.

Examples of the pharmacologically active substances as described in general as opioids are morphine, hydromorphone, oxymorphone and codeine. Morphine is the preferred substance. The substances can be used in the form of their salts, sick as alkali metal or amine salts or as acid addition salts; or as esters such as lower alkyl esters, or as solvates (hydrates), to optimise the activity, efficacy and/or stability of the substance. Morphine sulphate and morphine hydrochloride are the preferred salts to be used according to the invention.

The invention further relates to the use of an opioid as claimed in claim 5.

### EXPERIMENTAL PART

For dry powder inhalation systems the patient inspiratory effort through the device is the main force delivering and aerosolizing the formulation. Upon inspiration the agglomerates or aggregates, which are formed during processing, should break apart and present the drug as more or less discrete particles for inhalation into the lung.

In order to document the dispersion characteristics, as a function of the inhaled air flow rate, *in vitro* performance test with the use of a impinger are performed. The basic mechanism in this experiment is impaction and the apparatus consists of several stages. The stages represent parts of the respiratory tract. In this manner the powder aerosol is characterized, in the sense of particle size distribution, on the basis of the aerodynamic behaviour of particles. The respirable fraction of a powder is defined as the mass of the particles with a diameter less than 6,8 µm. This respirable fraction is reflected in the determination of the fine particle dose (in mg) or the fine particle fraction (% relative to the delivered dose, defined as the sum of all stages of a impinger and the throat).

The above characterization of a preparation meets the standards of the "Inhalanda" Monograph of the European Pharmacopeia, as published in Pharmeuropa 1996, p. 245-258.

### EXAMPLES

### Preparation of the mixtures

Morphine sulphate BP was micronized using an air jet mill (LS 100, GfM) at a pressure of 4 bar and a feed rate of 5 g/min. The particle size distribution was determined using a laser diffraction particle sizer (Malvern Mastersizer X). A mixture with lactose monohydrate was obtained by using a high-shear mixer (Robot Coupe R2) during 5 minutes. The ratio of morphine sulphate:lactose in the obtained mixture was 1:17. This mixture was used to fill the cartridges for the Cyclohaler (Example 1), to fill the Cyclovent (Example 3) and to fill the Disphaler (Example 5). All dosages weighted 25 mg. In addition pure micronized morphine sulphate was used to fill the cartridges for the Cyclohaler (Example 2), to fill the Cyclovent (Example 4) and to fill the Disphaler (Example 6). These dosages weighted 10 mg.

### Characterization of the aerosol formulations

For determination of the fine particle fraction all inhalation means were characterized by using a multi-stage liquid impactor (Copley, UK) made from glass and metal having four impaction stages and a filter (PA/PH/Exp. 12/T (96) 11 ANP). The nominal cut-off diameter of the stages is 13 µm, 6.8 µm, 3.1 µm and 1.7 µm at the operating air flow rate of 60 ± 5 litres per minute. A total volume of 4 litres of air was applied. In the tests with the Cyclohaler, 10 doses were sampled. However, in the tests with the Disphaler and Cyclovent 5 doses were sampled. All stages including the filter, the throat were analyzed on morphine sulphate by using a high performance liquid chromatography (HPLC) method. The HPLC method was performed by using a Symmetry C18 250 × 4.6 mm ID column (Waters, Milford, Massachusettes, USA), a mobile phase of acetonitrile:water (50:50) with 0.1 M sodium lauryl sulphate and 0.04 M potassium hydrogen phosphate dissolved in water, and a UV detector set at 287 nm. All samples were dissolved in acetonitrile:water (50:50). All calculations were related to morphine as a free base.

### EXAMPLE 1

| Cyclohaler | |
|---|---|
| | mg morphine |
| throat | 0,12 |
| stage 1 (< 13 µm) | 0,30 |
| stage 2 (< 6,8 µm) | 0,10 |
| stage 3 (< 3,1 µm) | 0,24 |
| stage 4 (< 1,7 µm) | 0,16 |
| filter | 0,04 |
| fine particle dose: 0,44 mg morphine | |
| fine particle fraction: 46 % (= respirable fraction; < 6,8 µm) | |

### EXAMPLE 2

| Cyclohaler | |
|---|---|
| | mg morphine |
| throat | 0,70 |
| stage 1 | 1,40 |
| stage 2 | 0,67 |
| stage 3 | 1,31 |
| stage 4 | 0,73 |
| filter | 0,29 |
| fine particle dose: 2,33 mg morphine | |
| fine particle fraction: 46 % | |

### EXAMPLE 3

| Cyclovent | |
|---|---|
| | mg morphine |
| throat | 0,20 |
| stage 1 | 0,26 |
| stage 2 | 0,10 |
| stage 3 | 0,23 |
| stage 4 | 0,17 |
| filter | 0,06 |
| fine particle dose: 0,46 mg morphine | |
| fine particle fraction: 45 % | |

### EXAMPLE 4

| Cyclovent | |
|---|---|
| | mg morphine |
| throat | 0,55 |
| stage 1 | 0,40 |
| stage 2 | 0,20 |
| stage 3 | 0,49 |
| stage 4 | 0,59 |
| filter | 0,50 |
| fine particle dose: 1,58 mg morphine | |
| fine particle fraction: 58 % | |

### EXAMPLE 5

| Disphaler | |
|---|---|
| | mg morphine |
| throat | 0,23 |
| stage 1 | 0,39 |
| stage 2 | 0,08 |
| stage 3 | 0,20 |
| stage 4 | 0,12 |
| filter | 0,04 |
| fine particle dose: 0,36 mg morphine | |
| fine particle fraction: 34 % | |

### EXAMPLE 6

| Disphaler | |
|---|---|
| | mg morphine |
| throat | 1,72 |
| stage 1 | 3,99 |
| stage 2 | 0,38 |
| stage 3 | 0,43 |
| stage 4 | 0,17 |
| filter | 0,11 |
| fine particle dose: 0,71 mg morphine | |
| fine particle fraction: 10 % | |

The formulation administered by the described means and according to the present invention shows good dispersion characteristics, as reflected by adequate fine particle doses. This indicates that various parts of the respiratory tract can be reached. Thus diseases and illnesses in these parts of the respiratory tract can be treated adequately. Furthermore, patients with poor lung function are able to inhale the formulations according to the invention and administered by the described modes.

## Claims

1. A pharmaceutical dry-powder composition suitable for inhalation consisting of micronized particles of an opioid having a fine particle fraction of at least 10%.

2. A pharmaceutical dry-powder composition according to claim 1, wherein said opioid is selected from the group consisting of morphine, hydromorphone, oxymorphone and codeine.

3. A pharmaceutical dry-powder composition according to claim 1 or 2, wherein said opioid is in de form of a salt, an ester or a solvate.

4. A pharmaceutical dry-powder composition according to claim 3, wherein said salt is an alkali metal salt, amine salt or an acid addition salt, said ester is a lower alkyl ester, and said solvate is a hydrate.

5. Use of an opioid having a fine particle fraction of at least 10% for the preparation of a dry powder inhalation medicament for the treatment of dyspnoea and pain.

## Patentansprüche

1. Pharmazeutische Trockenpulver-Zusammensetzung, die zur Inhalation geeignet ist und aus mikronisierten Teilchen eines Opioids mit einem Feinteilchenbruchteil von mindestens 10 % besteht.

2. Pharmazeutische Trockenpulver-Zusammensetzung nach Anspruch 1, bei der das Opioid ausgewählt ist aus der Gruppe bestehend aus Morphin, Hydromorphon, Oxymorphon und Codein.

3. Pharmazeutische Trockenpulver-Zusammensetzung nach Anspruch 1 oder 2, bei der das Opioid in Form eines Salzes, eines Esters oder eines Solvats vorliegt.

4. Pharmazeutische Trockenpulver-Zusammensetzung nach Anspruch 3, bei der das Salz ein Alkalimetallsalz, ein Aminsalz oder ein Säureadditionssalz ist, der Ester ein Niederalkylester ist und das Solvat ein Hydrat ist.

5. Verwendung eines Opioids mit einem Feinteilchenbruchteil von mindestens 10 % für die Herstellung eines Trockenpulver-Inhalationsmedikamentes für die Behandlung von Dyspnoe und Schmerz.

## Revendications

1. Composition pharmaceutique sèche pouvant être inhalée, laquelle est composée de particules micronisées d'un opioïde ayant un taux de particules fines d'au moins 10 %.

2. Composition pharmaceutique sèche selon la revendication 1, dans laquelle ledit opioïde est choisi dans le groupe constitué par la morphine, l'hydromorphone, l'oxymorphone et la codéine.

3. Composition pharmaceutique sèche selon la revendication 1 ou 2, dans laquelle ledit opioïde se trouve sous forme de sel, d'ester ou de solvate.

4. Composition pharmaceutique sèche selon la revendication 3, dans laquelle ledit sel est un sel métallique alcalin, un sel aminé ou un sel d'addition acide, ledit ester un ester d'alkyle avec chaîne courte et ledit solvate un hydrate.

5. Utilisation d'un opioïde ayant un taux de particules fines d'au moins 10 % en vue de la préparation d'un médicament à inhaler sous forme sèche destiné au traitement de la dyspnée et de la douleur.
